# EUROPEAN PATENT SPECIFICATION

(11) **EP 3 643 694 B1**
(45) Date of publication and mention of the grant of the patent: **15.11.2023**
(21) Application number: 18819739.6
(22) Date of filing: 20.06.2018
(51) Int. Cl.: C07C 17/269, C07C 21/20, C07C 22/08, B01J 31/24, B01J 37/16, B01J 23/06, B01J 23/34, B01J 31/18, B01J 31/22

(54) **METHOD FOR PRODUCING BUTADIENE COMPOUND**
VERFAHREN ZUR HERSTELLUNG EINER BUTADIENVERBINDUNG
PROCÉDÉ DE PRODUCTION DE COMPOSÉ DE BUTADIÈNE

(30) Priority: 20.06.2017 JP 2017120576
(43) Date of publication of application: 29.04.2020
(73) Proprietor: Daikin Industries, Ltd., Osaka-shi, Osaka 530-0001 (JP)
(72) Inventor: GOTOU, Akihiro, Osaka-shi Osaka 530-0001 (JP); HOSHIYA, Naoyuki, Osaka-shi Osaka 530-0001 (JP); HIGASHI, Masahiro, Osaka-shi Osaka 530-0001 (JP); HAMADA, Tomohito, Osaka-shi Osaka 530-0001 (JP); KISHIKAWA, Yosuke, Osaka-shi Osaka 530-0001 (JP)
(74) Representative: Hoffmann Eitle
(86) International application number: PCT/JP2018/023534
(87) International publication number: WO 2018/235883

(56) References cited:
- CN-A- 106 495 982
- CN-A- 106 495 982
- JP-A- H01 224 331
- JP-A- H01 224 331
- JP-A- S50 112 307
- JP-A- 2012 067 068
- JP-A- 2012 067 068
- IVAN WLASSICS ET AL: "Coupling Reactions of Chlorofluoro and Perfluoroalkyl Iodides", COLLECTION OF CZECHOSLOVAK CHEMICAL COMMUNICATIONS, vol. 73, no. 12, 2008, pages 1719-1728, XP055770766, CZ ISSN: 0010-0765, DOI: 10.1135/cccc20081719 ISBN: 978-80-86241-25-8
- Anonymous: "JIKKYO Science materials", Jikkyo shuppan co , 22 March 2011 (2011-03-22), pages 1-5, XP055658118, Retrieved from the Internet: URL:http://www.jikkyo.co.jp/download/detai l/45/9992655101

## Description

### Technical Field

The present invention relates to a butadiene compound.

### Background Art

Butadiene compounds are industrially important compounds used for various applications, such as synthetic rubber starting materials, non-linear optical materials, liquid crystal compound materials, and dye starting materials.

Accordingly, a method for easily obtaining a butadiene compound with low cost and high yield has been studied.

JP-A-2012-67068, for example, provides a method for producing hexafluoro-1,3-butadiene from tetrafluoroethylene by using an organic metal compound in the presence of a transition metal catalyst.

However, in particular, the method for synthesizing a fluorine-containing butadiene compound is limited, and an easy and efficient butadiene compound production method is further demanded.

CN-A-106495982 discloses a method for preparing hexafluoro-1,3-butadiene which involves a step of coupling CF₂=CFBr using Fe-chloride or Cu-chloride as a Catalyst in the presence of zinc as reducing agent.
I. Wlassics et al., Collect. Czech. Chem. Commun., 73(12), 1719-28 (2008) describes the formation of hexafluoro-1,3-butadiene as a by-product in the Cu-catalyzed coupling of CF₂=CFBr in the presence of phenyl iodide as reducing agent.

### Summary of Invention

### Technical Problem

In view of the above problem, an object of the present invention is to provide a method for easily and efficiently producing a butadiene compound.

### Solution to Problem

As a result of extensive research, the present inventors found that the following method can solve the above problem:
A method for producing a compound of formula (1): 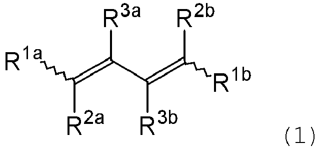 wherein R^{1a}, R^{1b}, R^{2a}, R^{2b}, R^{3a}, and R^{3b} each independently are H, F, alkyl, fluoroalkyl or aryl; provided that at least one of them is F or fluoroalkyl; or one or more moieties among R^{1a}-C-R^{2a}, R^{1b}-C-R^{2b}, R^{1a}-C=C-R^{3a} and R^{1b}-C=C-R^{3b} each independently may form a non-aromatic carbon ring that may be substituted with one or more fluorine atoms; and
each single bond expressed by a wavy line shows that the configuration with respect to a double bond to which the single bond is bonded is E configuration, Z configuration, or a mixture thereof at any ratio;
the method comprising step A of subjecting a compound of formula (2a) : 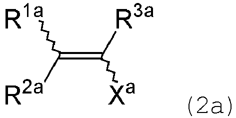 wherein X^{a} is Cl, Br or I, and R^{1a}-R^{3a} are as defined above, and a compound of formula (2b): 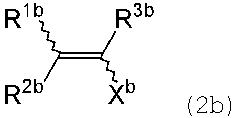 wherein X^{b} is Cl, Br or I, and R^{1b}-R^{3b} are as defined above, to a coupling reaction in the presence of
   (1) a metal catalyst which is at least one of nickel catalysts, palladium catalysts, platinum catalysts, ruthenium catalysts, rhodium catalysts, and cobalt catalysts, and
   (2) one or more reducing agents selected from (a) transition metal elements in the 4th period, (b) metal elements in group 12, (c) metal elements in group 13 and (e) carbon monoxide.

Preferred embodiments of the invention are as defined in the appended dependent claims and/or in the following detailed description.

### Advantageous Effects of Invention

The present invention provides a method for easily and efficiently producing a butadiene compound (also referred to as "the present method" hereinafter).

According to the present method, a butadiene compound can be produced from an easily available starting material (e.g., 1-chloro-1-fluoroethylene and chlorotrifluoroethylene) through a one-step reaction.

In the production of a compound using metal, disposal of the metal is sometimes a typical problem. In one embodiment of the present method, a target compound can be obtained with a small amount of metal, and thus the present invention is excellent.

The present method is excellent because a target compound can be obtained using an easily available solvent without the need for a large amount of a special solvent.

### Description of Embodiments

### Terms

The symbols and the abbreviations in this specification can be interpreted as having the general meanings in the technical field to which the present invention pertains, according to the context of this specification, unless otherwise specified. Especially, in the present specification the following applies.

The term "comprise" or "contain" is intended to encompass the meanings of "consist essentially of" and "consist of."

The steps, treatments, or operations in this specification can be performed at room temperature unless otherwise specified. Room temperature refers to a temperature of 10-35°C.

Unless otherwise specified, examples of the "alkyl group" include linear or branched C₁₋₁₀-alkyl, such as methyl, ethyl, propyl (e.g., n-propyl and isopropyl), butyl (e.g., n-butyl, isobutyl, sec-butyl, and tert-butyl), pentyl (e.g., n-pentyl, tert-pentyl, neopentyl, isopentyl, sec-pentyl, and 3-pentyl), hexyl, heptyl, octyl, nonyl, and decyl.

The term "Cn-m" (herein, n and m are numbers) indicates that the carbon number is n or more and m or less, as would be usually understood by a person skilled in the art.

Unless otherwise specified, the "aryl" group may be monocyclic, dicyclic, tricyclic, or tetracyclic.

Unless otherwise specified, examples of the "aryl group" include C₆₋₁₈ aryl groups.

In this specification, unless otherwise specified, examples of the "aryl group" include phenyl, 1-naphthyl, 2-naphthyl, 2-biphenyl, 3-biphenyl, 4-biphenyl, and 2-anthryl.

The "fluoroalkyl group" is an alkyl group having at least one H replaced by F.

The number of fluorine atoms in the "fluoroalkyl group" may be one or more (e.g., 1-3, 1-6, 1-12, or 1 to the maximum replaceable number).

The "fluoroalkyl group" encompasses perfluoroalkyl, and "perfluoroalkyl" is an alkyl group having all H replaced by F.

The "fluoroalkyl group" may be a fluoroalkyl group having a carbon number of 1-20, 1-12, 1-6, 1-4, 1-3, 6, 5, 4, 3, 2, or 1.

The "fluoroalkyl group" may be linear or branched.

Specific examples of the "fluoroalkyl group" include fluoromethyl, difluoromethyl, trifluoromethyl, 2,2,2-trifluoroethyl, pentafluoro ethyl, tetrafluoropropyl (e.g., HCF₂CF₂CH₂-), hexafluoro propyl (e.g., (CF₃)₂CH-), nonafluorobutyl, octafluoropentyl (e.g., HCF₂CF₂CF₂CF₂CH₂-) and tridecafluorohexyl.

### Production Method

The present method is a method for producing a compound of formula (1): 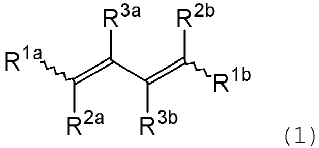 wherein R^{1a}, R^{1b}, R^{2a}, R^{2b}, R^{3a}, and R^{3b} each independently are H, F, alkyl, fluoroalkyl or aryl; provided that at least one of them is F or fluoroalkyl; or one or more moieties among R^{1a}-C-R^{2a}, R^{1b}-C-R^{2b}, R^{1a}-C=C-R^{3a} and R^{1b}-C=C-R^{3b} each independently may form a non-aromatic carbon ring that may be substituted with one or more fluorine atoms; and
each single bond expressed by a wavy line shows that the configuration with respect to a double bond to which the single bond is bonded is E configuration, Z configuration, or a mixture thereof at any ratio;
the method comprising step A of subjecting a compound of formula (2a) : 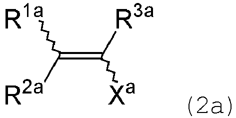 wherein X^{a} is Cl, Br or I, and R^{1a}-R^{3a} are as defined above, and a compound of formula (2b): 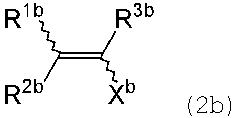 wherein X^{b} is Cl, Br or I, and R^{1b}-R^{3b} are as defined above, to a coupling reaction in the presence of
   (1) a metal catalyst which is at least one of nickel catalysts, palladium catalysts, platinum catalysts, ruthenium catalysts, rhodium catalysts, and cobalt catalysts, and
   (2) one or more reducing agents selected from (a) transition metal elements in the 4th period, (b) metal elements in group 12, (c) metal elements in group 13 and (e) carbon monoxide.

In one preferable embodiment of the present method, in formula (1) and correspondingly in the formulae (2a) and (2b), at least one of R^{1a}, R^{1b}, R^{2a}, R^{2b}, R^{3a}, and R^{3b} is F or fluoroalkyl; or
either or both of R^{1a}-C-R^{2a} moiety and R^{1b}-C-R^{2b} each may form a non-aromatic carbon ring that may be substituted with one or more fluorine atoms.

In one preferable embodiment of the present method, in formula (1), and correspondingly in the formulae (2a) and (2b),
R^{1a} is H, F, alkyl, fluoroalkyl or aryl,
R^{2a} is H, F or fluoroalkyl;
   or R^{1a}-C-R^{2a} may form a non-aromatic carbon ring that may be substituted with one or more fluorine atoms;
R^{3a} is F or fluoroalkyl;
R^{1b} is H, F, alkyl, fluoroalkyl or aryl, and
R^{2b} is H, F or fluoroalkyl;
   or R^{1b}-C-R^{2b} may form a non-aromatic carbon ring that may be substituted with one or more fluorine atoms; and
R^{3b} is F or fluoroalkyl.

Examples of the "non-aromatic hydrocarbon ring" in the "non-aromatic carbon ring that may be substituted with one or more fluorine atoms" that may be formed by the R^{1a}-C-R^{2a} moiety or the R^{1b}-C-R^{2b} include C₃₋₈ non-aromatic hydrocarbon rings. Specific examples include
(1) C₃₋₈ cycloalkanes, such as cyclopropane, cyclobutane, cyclopentane, cyclohexane, cycloheptane, and cyclooctane;
(2) C₅₋₈ cycloalkenes, such as cyclopentene, cyclohexene, cyclohepten, and cyclooctene;
(3) C₅₋₈ cycloalkadienes, such as cyclopentadiene, cyclohexadiene, cycloheptadiene, and cyclooctadiene; and
(4) C₅₋₈ bridged cyclic hydrocarbons, such as bicyclo[2.1.0]pentane, bicyclo[2.2.1]heptane, bicyclo[3.2.1]octane, bicyclo[2.2.1]hept-2-ene, and tricyclo[2.2.1.0]heptane.

Examples of the "non-aromatic hydrocarbon ring" in the "non-aromatic carbon ring that may be substituted with one or more fluorine atoms" that may be formed by the R^{1a}-C=C-R^{3a} moiety or R^{1b}-C=C-R^{3b} moiety include C₃₋₈ (preferably C₅₋₈) non-aromatic unsaturated hydrocarbon rings. Specific examples include:
(1) C₅₋₈ cycloalkenes, such as cyclopentene, cyclohexene, cycloheptene, and cyclooctene; and
(2) C₅₋₈ cycloalkadienes, such as cyclopentadiene, cyclohexadiene, cycloheptadiene, and cyclooctadiene.

The following is described just to be sure. Of R^{1a}, R^{1b}, R^{2a}, R^{2b}, R^{3a}, and R^{3b}, groups that do not involve the above ring formation can be understood by referring to the explanation of each group in the specification.

The number of fluorine atoms that may be contained in the "non-aromatic hydrocarbon ring" may be one to the maximum replaceable number.

The following is described just to be sure. As would be usually understood by a person skilled in the art, a non-aromatic hydrocarbon ring replaced by the maximum replaceable number of fluorine atoms does not have a hydrogen atom.

R^{1a} is preferably H, F, fluoroalkyl, alkyl or aryl; more preferably H, F, trifluoromethyl or aryl; and even more preferably H or F.

R^{2a} is preferably H, F, fluoroalkyl, alkyl or aryl; more preferably H, F or trifluoromethyl; and even more preferably H or F.

R^{3a} is preferably H, F, fluoroalkyl, alkyl or aryl; more preferably H, F or trifluoromethyl; and even more preferably F or trifluoromethyl.

R^{1b} is preferably H, F, fluoroalkyl, alkyl or aryl; more preferably H, F, trifluoromethyl or aryl; and even more preferably H or F.

R^{2b} is preferably H, F, fluoroalkyl, alkyl or aryl; more preferably H, F or trifluoromethyl, and even more preferably H or F.

R^{3b} is preferably H, F, fluoroalkyl, alkyl or aryl; more preferably H, F or trifluoromethyl; and even more preferably F or trifluoromethyl.

However, as described above, at least one of R^{1a}, R^{1b}, R^{2a}, R^{2b}, R^{3a}, and R^{3b} is F or fluoroalkyl.

When the compound of formula (2a) is different from the compound of formula (2b), the following [1], [2], and [3] can be obtained by the reaction of step A.
[1] a compound obtained by heterocoupling of the compound of formula (2a) and the compound of formula (2b),
[2] a compound obtained by homocoupling of two molecules of the compound of formula (2a), and
[3] a compound obtained by homocoupling of two molecules of the compound of formula (2b).

These compounds can be isolated or purified by a conventional method if desired.

In one preferable embodiment of the present invention, the compounds of formulae (2a) and (2b) are the same.

In the embodiment, the reaction of step A is homocoupling of the compound of formula (2a) (or compound of formula (2b)).

X^{a} is preferably Cl or Br, and particularly preferably Cl.

X^{b} is preferably Cl or Br, and particularly preferably Cl.

### Metal catalyst

The metal catalyst used in the present invention is a transition metal catalyst which is at least one of nickel catalysts, palladium catalysts, platinum catalysts, ruthenium catalysts, rhodium catalysts, and cobalt catalyst. Of these, preferred is a transition metal catalyst in which the transition metal is palladium or nickel. Specifically, the metal catalyst is preferably at least one of palladium catalysts and nickel catalysts.

Examples of transition metal catalysts in which the transition metal is palladium include zerovalent palladium complexes; zerovalent palladium complexes generated from monovalent or divalent palladium complexes during a reaction; and complexes obtained by mixing these palladium complexes with at least one compound (ligand) selected from the group consisting of ketones, diketones, phosphines, diamines, bipyridines, and phenanthrolines.

Examples of zerovalent palladium complexes include, but are not limited to, Pd₂(dba)₃ (dba is dibenzylideneacetone), Pd₂(dba)₃-CHCl₃, Pd(dba)₂, Pd(cod)₂ (cod is cycloocta-1,5-diene), Pd(dppe)₂ (dppe is 1,2-bis(diphenylphosphino)ethane), Pd(PCy₃)₂ (Cy is cyclohexyl), Pd(Pt-Bu₃)₂ (t-Bu is t-butyl), Pd(PPh₃)₄ (Ph is phenyl), tris{tris [3,5-bis(trifluoromethyl)phenyl]phosphine}palladium (0), etc.

Examples of monovalent palladium complexes include palladium complexes of the following chemical formula: 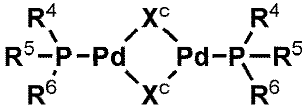 wherein X^{c} is Cl, Br or I; R⁴, R⁵, and R⁶ each independently at each occurrence are C₁₋₂₀-alkyl, alkenyl, alkynyl, or aryl.

Specifically, preferable examples include di-µ-chlorobis(tri-tert-butylphosphine)dipalladium (I), di-µ-bromobis(tri-tert-butylphosphine)dipalladium (I), di-µ-iodobis(tri-tert-butylphosphine)dipalladium (I), di-µ-chlorobis{tri(1-adamanthyl)phosphine}dipalladium (I), di-µ-bromobis{tri(1-adamanthyl)phosphine}dipalladium (I) and di-µ-iodobis{tri(1-adamanthyl)phosphine}dipalladium (I).

Examples of divalent palladium complexes include palladium chloride, palladium bromide, palladium acetate, bis(acetylacetonato)palladium (II), dichloro(η⁴-1,5-cyclooctadiene) palladium (II), dibromo(η⁴-1,5-cyclooctadiene) palladium (II), bis(acetonitrile)dichloropalladium (II), bis(benzonitrile)dichloropalladium (II), and di-p-chlorobis{(η-allyl)palladium} (II); and complexes obtained by binding a phosphine ligand, such as triphenylphosphine, to these complexes.

These divalent palladium complexes are, for example, reduced by a reducing species (e.g., phosphines, reducing agents, and organic metal reagents) that is co-present during a reaction, thereby generating zerovalent palladium complexes.

The above zerovalent palladium complexes or zerovalent palladium complexes generated from monovalent or divalent palladium complexes through reduction can interact with a compound (ligand), such as ketones, diketones, phosphines, diamines, bipyridines, and phenanthroline optionally added during a reaction, and can be converted into zerovalent palladium complexes that are involved in the reaction. It is not always necessary to know how many ligands are bound to a zerovalent palladium complex during the reaction.

Using the above ligands, these palladium complexes are often formed into a homogeneous solution with a reaction substrate to be used in the reaction. In addition, these palladium complexes can also be used as a heterogeneous catalyst dispersed or supported in a polymer such as polystyrene and polyethylene. Such heterogeneous catalysts have an advantage in a process such as a catalyst recovering process. Specific examples of catalyst structures include those in which a metal atom is immobilized by a polymeric phosphine that is a crosslinked polystyrene chain having a phosphine introduced thereto, as shown in the following chemical formula:

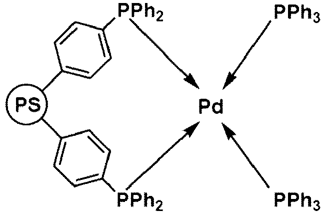

The "metal catalyst" used in the present invention may be a supported catalyst in which a transition metal is supported by a carrier. Such a supported catalyst has a cost advantage because the catalyst can be recycled.

Examples of carriers include carbon, alumina, silica gel alumina, silica gel, barium carbonate, barium sulfate, calcium carbonate, titanium oxide, zirconium oxide, calcium fluoride, and zeolite.

In addition, polymeric phosphines disclosed in Kanbara et al., Macromolecules, 33, 657 (2000); Yamamoto et al., J. Polym. Sci., 40, 2637 (2002); JP-A-1994-032763; JP-A-2005-281454 and JP-A-2009-527352 can also be used.

Herein, ketone is not limited, and dibenzylideneacetone is one example.

Examples of diketones include ***β***-diketones, such as acetylacetone, 1-phenyl-1,3-butanedione, 1,3-diphenylpropanedione and hexafluoroacetylacetone.

Since phosphines having a halogen-phosphorus bond per se may be subjected to a coupling reaction, phosphines are preferably dialkyl monoaryl phosphines, diaryl monoalkyl phosphines, trialkylphosphines, triarylphosphines, or bidentate diphosphines.

Specific examples of dialkyl monoaryl phosphines include diisopropylphenyl phosphine, diisopropyl(o-tolyl) phosphine, diisopropyl(2,6-dimethylphenyl)phosphine, diisopropyl pentafluorophenyl phosphine, di-n-butylphenyl phosphine, di-n-butyl(o-tolyl)phosphine, di-n-butyl(2,6-dimethylphenyl)phosphine, di-n-butyl pentafluorophenyl phosphine, di-tert-butylphenyl phosphine, di-tert-butyl(o-tolyl)phosphine, di-tert-butyl(2,6-dimethylphenyl)phosphine, di-tert-butyl pentafluorophenyl phosphine, dicyclohexyl phenylphosphine, dicyclohexyl(o-tolyl)phosphine, dicyclohexyl(2,6-dimethylphenyl)phosphine, dicyclohexyl pentafluorophenyl phosphine, di(1-adamanthyl)phenylphosphine, di(1-adamanthyl) (o-tolyl)phosphine, di(1-adamanthyl) (2,6-dimethylphenyl)phosphine, di(1-adamanthyl)pentafluorophenyl phosphine, 2-dicyclohexylphosphino-2',6'-diisopropoxy biphenyl, 2-dicyclohexylphosphino-2',6'-dimethoxy biphenyl, 2-dicyclohexylphosphino-2',4',6'-triisopropyl biphenyl, 2-dicyclohexylphosphino-2'-(N,N-dimethylamino)biphenyl, 2-(dicyclohexylphosphino)-3,6-dimethoxy-2',4',6'-triisopropyl-1,1'-biphenyl, 2'-dicyclohexylphosphino-2,4,6-trimethoxybiphenyl, 2-dicyclohexyl phosphino-2'-methylbiphenyl, 2-di-tert-butylphosphino-2'-methylbiphenyl, 2-di-tert-butylphosphino-2'-(N,N-dimethylamino)biphenyl, 2-di-tert-butylphosphino-3,4,5,6-tetramethyl-2',4',6'-triisopropyl-1,1'-biphenyl, 2-(di-tert-butylphosphino)-2',4',6'-triisopropyl-3,6-dimethoxy-1,1'-biphenyl, 2-di-tert-butylphosphino-2',4',6'-triisopropyl biphenyl, (2-biphenyl)dicyclohexylphosphine, and (2-biphenyl)di- tert-butylphosphine.

Specific examples of diarylmonoalkylphosphines include diphenylmethylphosphine, diphenylisopropylphosphine, n-butyl diphenylphosphine, tert-butyl diphenylphosphine, cyclohexyl diphenylphosphine, (1-adamanthyl)diphenylphosphine, di(o-tolyl)methylphosphine, di(o-tolyl)isopropylphosphine, n-butyldi(o-tolyl)phosphine, tert-butyldi(o-tolyl)phosphine, cyclohexyldi(o-tolyl)phosphine, (1-adamanthyl)di(o-tolyl)phosphine, bis(2,6-dimethylphenyl)methylphosphine, bis(2,6-dimethylphenyl)isopropylphosphine, bis(2,6-dimethylphenyl)-n-butylphosphine, bis(2,6-dimethylphenyl)-tert-butylphosphine, bis(2,6-dimethylphenyl)cyclohexylphosphine, (1-adamanthyl)bis(2,6-dimethylphenyl)phosphine, bis(pentafluorophenyl)methylphosphine, bis(pentafluorophenyl)isopropylphosphine, bis(pentafluorophenyl)-n-butylphosphine, bis(pentafluorophenyl)-tert-butylphosphine, bis(pentafluorophenyl)cyclohexylphosphine and(1-adamanthyl)bis(pentafluorophenyl)phosphine.

Specific examples of trialkylphosphines include tri(C₃₋₂₀ alkyl)phosphines, such as tricyclohexylphosphine, triisopropylphosphine, tri-tert-butylphosphine, trihexylphosphine, tri(1-adamanthyl)phosphine, tricyclopentylphosphine, di-tert-butyl methylphosphine, cyclohexyldi-tert-butylphosphine, di-tert-butyl neopentylphosphine, di-tert-butyl isopropylphosphine, di-tert-butyl(2-butenyl)phosphine, di-tert-butyl(3-methyl-2-butenyl)phosphine, 1-adamanthyl-di-tert-butylphosphine, tert-butyldi(1-adamanthyl)phosphine, di(1-adamanthyl)isopropylphosphine, cyclohexyldi(1-adamanthyl)phosphine, n-butyldi(1-adamanthyl)phosphine, tribicyclo[2,2,2]octylphosphine and trinorbornyl phosphine.

Specific examples of triarylphosphines include tri(monocyclic aryl)phosphines, such as triphenylphosphine, trimesitylphosphine, tri(o-tolyl)phosphine, tris{(4-trifluoromethyl)phenyl}phosphine, tris(pentafluorophenyl)phosphine and tris[3,5-bis(trifluoromethyl)phenyl]phosphine.

Specific examples of bidentate diphosphines include 1,2-bis(diphenylphosphino)ethane, 1,3-bis(diphenylphosphino)propane, 1,4-bis(diphenylphosphino)butane, 1,5-bis(diphenylphosphino)pentane, 1,3-bis(diisopropylphosphino)propane, 1,4-bis(diisopropylphosphino)butane, 1,3-bis(dicyclohexylphosphino)propane, 1,4-bis(dicyclohexylphosphino)butane, bis(diphenylphosphinophenyl)ether, bis(dicyclohexylphosphinophenyl)ether, 1,1'-bis(diphenylphosphino)ferrocene, 1,1'-bis(dicyclohexylphosphino)ferrocene, 1,1'-bis(diisopropylphosphino)ferrocene, 1,1'-bis(di-tert-butyl phosphino)ferrocene, 1,2-bis(di-tert-butylphosphino methyl)benzene, 4,6-bis(diphenylphosphino)phenoxazine, 4,5-bis(diphenylphosphino)-9,9'-dimethylxanthene, 4,5-bis(di-tert-butylphosphino)-9,9'-dimethylxanthene and 2,2'-bis(diphenylphosphino)-1,1'-binaphthyl.

Phosphines used in the present invention may be tetrafluoro borates (e.g., trialkylphosphonium tetrafluoroborates, such as trihexylphosphonium tetrafluoroborate and tri-tert-butyl phosphonium tetrafluoroborate). Such a salt can be reacted with a base to give a free body of phosphine (e.g., trialkylphosphine, such as tricyclohexyl phosphine and tri-tert-butylphosphine).

Examples of the base include amines, inorganic bases, organic metal bases.

Examples of amines include triethylamine, tri(n-propyl)amine, tri(n-butyl)amine, diisopropylethylamine, cyclohexyldimethylamine, pyridine, lutidine, γ-collidine, *N,*N-dimethylaniline, *N*-methylpiperidine, *N*-methylpyrrolidine, *N-*methylmorpholine, 1,8-diazabicyclo[5,4,0]-7-undecene, 1,5-diazabicyclo[4,3,0]-5-nonene, and 1,4-diazabicyclo[2,2,2]octane.

Examples of inorganic bases include lithium hydroxide, potassium hydroxide, sodium hydroxide, calcium hydroxide, lithium carbonate, sodium carbonate, potassium carbonate, sodium hydrogen carbonate, potassium hydrogen carbonate, lithium phosphate, sodium phosphate, and potassium phosphate.

Examples of organic metal bases include organic alkali metal compounds, such as butyllithium, tert-butyllithium, phenyllithium, sodium triphenylmethyl, and sodium ethyl; organic alkali earth metal compounds, such as methylmagnesium bromide, dimethylmagnesium, phenylmagnesium chloride, phenylcalcium bromide, and bis(dicyclopentadiene)calcium; and alkoxides, such as sodium methoxide and tert-butyl methoxide.

Preferable examples of bases include triethylamine, potassium carbonate, and potassium phosphate. More preferable examples of bases include potassium carbonate and potassium phosphate. Particularly preferable examples of bases include potassium phosphate.

The bases can be used singly or in a combination of two or more.

Phosphines used in the present invention may be in the oxide form.

Examples of the oxide form include di(cyclo)alkylphosphine oxides (e.g., di-tert-butylphosphine oxide and di(1-adamanthyl)phosphine oxide).

As stated above, aryl phosphines for a heterogeneous catalyst in which phosphine units are incorporated into a polymer chain can also be preferably used. A specific example is a triarylphosphine formed by binding one of the phenyl groups of triphenylphosphine to a polymer chain as shown in the chemical formula below:

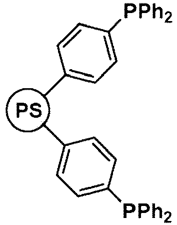

Examples of diamines include tetramethylethylenediamine and 1,2-diphenylethylenediamine.

Examples of bipyridines include 2,2'-bipyridyl, 4,4'-dimethyl-2,2'-bipyridyl, 5,5'-dimethyl-2,2'-bipyridyl, 6,6'-dimethyl-2,2'-bipyridyl, 4,4'-di-tert-butyl-2,2'-bipyridine, 4,4'-dimethoxy-2,2'-bipyridyl, 2,2'-biquinoline and α,α',α" - tripyridyl.

Examples of phenanthrolines include 1,10-phenanthroline, 2-methyl-1,10-phenanthroline, 3-methyl-1,10-phenanthroline, 5-methyl-1,10-phenanthroline, 2,9-dimethyl-1,10-phenanthroline, 2,9-diphenyl-1,10-phenanthroline, 4,7-dimethyl- 1,10-phenanthroline, 5,6-dimethyl-1,10-phenanthroline, 4,7-diphenyl-1,10-phenanthroline, 2,9-dimethyl-4,7-diphenyl-1,10-phenanthroline and 3,4,7,8-tetramethyl-1,10-phenanthroline.

Among these ligands, ligands of phosphines, diamines, bipyridines, and phenanthrolines are preferred; and triarylphosphines and trialkylphosphine are more preferred. Preferable examples of triarylphosphines include triphenylphosphine and tris[3,5-bis(trifluoromethyl)phenyl]phosphine; and preferable examples of trialkylphosphines include tricyclohexylphosphine, tri-tert-butylphosphine, triisopropylphosphine, and tri(1-adamanthyl)phosphine.

Similarly, as described above, a triarylphosphine formed by binding one of the phenyl groups of triphenylphosphine to a polymer chain is also preferred.

Examples of transition metal catalysts in which the transition metal is nickel include zerovalent nickel complexes; zerovalent nickel complexes generated from divalent nickel complexes during a reaction; and complexes obtained by mixing these nickel complexes with at least one compound (ligand) selected from the group consisting of ketones, diketones, phosphines, diamines, bipyridines, and phenanthrolines.

Examples of zerovalent nickel complexes include Ni(cod)₂, Ni(cdd)₂ (cdd is cyclodeca-1,5-diene), Ni(cdt)₂ (cdt is cyclodeca-1,5,9-trien), Ni(vch)₂ (vch is 4-vinylcyclohexene), Ni(CO)₄, (PCy₃)₂Ni-N≡N-Ni(PCy₃)₂, and Ni(PPh₃)₄.

Examples of divalent nickel complexes include nickel chloride, nickel bromide, nickel acetate, bis(acetylacetonato)nickel(II), and complexes obtained by binding a ligand of phosphine, such as triphenylphosphine, to these complexes. These divalent nickel complexes are, for example, reduced by a reducing species (e.g., phosphines, zinc, and organic metal reagents) that is co-present during a reaction, thereby generating zerovalent nickel complexes.

The above zerovalent nickel complexes or zerovalent nickel complexes generated from divalent nickel complexes through reduction can interact with a ligand that is optionally added during a reaction, and can be converted into zerovalent nickel complexes that are involved in the reaction. It is not always necessary to know how many ligands are bound to a zerovalent nickel complex during the reaction.

Using the above ligands, these nickel complexes are often formed into a homogeneous solution with a reaction substrate and used in the reaction. In addition, these nickel complexes can also be used as a heterogeneous catalyst dispersed or supported in a polymer such as polystyrene and polyethylene. Such heterogeneous catalysts have an advantage in a process such as a catalyst recovering process. Specific examples of catalyst structures include those in which a metal atom is immobilized by a polymeric phosphine that is a crosslinked polystyrene chain having a phosphine introduced thereto, as shown in the following chemical formula.

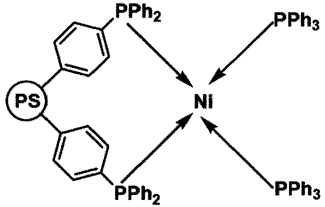

In addition, polymeric phosphines disclosed in Kanbara et al., Macromolecules, 33, 657 (2000); Yamamoto et al., J. Polym. Sci., 40, 2637 (2002); JP-A-1994-032763; JP-A-2005-281454 and JP-A-2009-527352 can also be used.

Examples of ketones, diketones, phosphines, diamines, bipyridines, and phenanthrolines are the same as those listed in the section on palladium complexes above.

As stated above, aryl phosphines for a heterogeneous catalyst in which phosphine units are incorporated into a polymer chain can also be preferably used. A specific example is a triarylphosphine formed by binding one of the phenyl groups of triphenylphosphine to a polymer chain as shown in the chemical formula below:

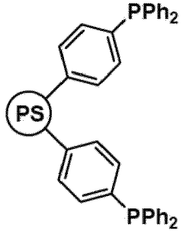

Among these ligands, ligands of phosphines, diamines, bipyridines, and phenanthrolines are preferred; and triarylphosphines and trialkylphosphine are more preferred. Preferable examples of triarylphosphines include triphenylphosphine, and preferable examples of trialkylphosphines include tricyclohexylphosphine, tri-tert-butylphosphine, triisopropylphosphine and tri(1-adamanthyl)phosphine.

Similarly, as described above, a triarylphosphine formed by binding one of the phenyl groups of triphenylphosphine to a polymer chain is also preferred.

Additionally, examples of platinum-containing catalysts include Pt(PPh₃)₄, Pt(cod)₂, Pt(dba)₂, platinum chloride, platinum bromide, bis(acetylacetonato)platinum (II), dichloro(η⁴-1,5-cyclooctadiene)platinum (II), or complexes obtained by binding a phosphine ligand, such as triphenylphosphine, to these catalysts; examples of ruthenium-containing catalysts include (Cl)₂Ru(PPh₃)₃, Ru(cot)(cod) (cot is cycloocta-1,3,5-trien), ruthenium chloride (III), dichloro (η⁴-1,5-cycloacetyloctadiene)ruthenium (II), tris(acetylacetonato)ruthenium (III), or complexes obtained by binding a phosphine ligand, such as triphenylphosphine, to these catalysts; examples of rhodium-containing catalysts include (Cl)Rh(PPh₃)₃, rhodium chloride (III), chloro(η⁴-1,5-cyclooctadiene)rhodium (I) dimer, tris(acetylacetonato)rhodium (III), or complexes obtained by binding a phosphine ligand, such as triphenylphosphine, to these catalysts; examples of cobalt-containing catalysts include (Cl)Co(PPh₃)₃, (C₅H₅)₂Co(PPh₃)₂(C₅H₅ is a cyclopentadienyl group), (C₅H₅)₂Co(cod)₂, tris(acetylacetonato)cobalt (III), cobalt(II) chloride, or complexes obtained by binding a phosphine ligand, such as triphenylphosphine, to these catalysts.

Of the catalysts, from the viewpoints of reactivity, yield, and selectivity, nickel- or palladium-containing catalysts, and particularly palladium-containing catalysts are preferred. Further, palladium complexes, particularly zerovalent palladium phosphine complexes (especially, a triaryl phosphine complex, diaryl monoalkyl phosphine complex, dialkyl monoaryl phosphine complex, trialkylphosphine complex, or a polymer phosphine complex of the following chemical formula) are preferred.

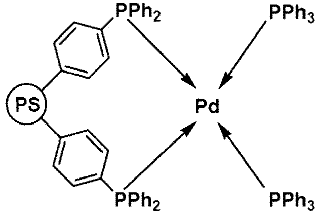

The molar ratio of the ligand/catalyst in the metal catalyst is, for example, 0.5-10, 1-10, 1-8, 1-6, 1-4, or 1-2.

The metal catalysts can be used singly or in a combination of two or more.

The upper limit of the amount of the metal catalyst is preferably 0.1 mol, more preferably 0.05 mol, and even more preferably 0.025 mol, per mol of the total amount of the compounds (2a) and (2b).

The lower limit of the amount of the metal catalyst is preferably 0.01 mol, more preferably 0.005 mol, and even more preferably 0.001 mol, per mol of the total amount of the compounds (2a) and (2b).

The amount of the metal catalyst is preferably 0.001-0.1 mol, more preferably 0.005-0.05 mol, and even more preferably 0.01-0.025 mol, per mol of the total amount of the compounds (2a) and (2b).

### Reducing agent

The reducing agent used in the present invention is an inorganic reducing agent, and is one or more selected from (a) transition metal elements in the 4th period, (b) metal elements in group 12, (c) metal elements in group 13 and (e) carbon monoxide. Preferable examples include elementary metal and carbon monoxide.

The metal as the reducing agent include transition metal elements in the 4th period (e.g., scandium, titanium, vanadium, chromium, manganese, iron, cobalt, nickel and copper); metal elements in group 12 (e.g., zinc, cadmium and mercury); and metal elements in group 13 (e.g., aluminum, gallium and indium).

Preferable examples of metal as the inorganic reducing agent include those having a standard electrode potential (oxidation reduction potential) in an aqueous solution within a range of -1.70 to -0.25, and those having a standard electrode potential (oxidation reduction potential) in an aqueous solution within a range of -1.20 to -0.3 are more preferred.

More preferable examples of metal as the reducing agent include manganese, iron, zinc, aluminum and indium, even more preferably zinc, manganese and indium.

The metal can be, for example, in a solid form, such as powder.

The reducing agents can be used singly or in a combination of two or more.

The upper limit of the amount of the reducing agent is preferably 10 mol, more preferably 7.5 mol, and even more preferably 5 mol, per mol of the total amount of the compounds (2a) and (2b).

The lower limit of the amount of the reducing agent is preferably 0.5 mol, more preferably 1 mol, and even more preferably 1.5 mol, per mol of the total amount of the compounds (2a) and (2b).

The amount of the reducing agent is preferably 0.5-10 mol, more preferably 1-7.5 mol, and even more preferably 1.5-5 mol, per mol of the total amount of the compounds (2a) and (2b).

In the reaction of step A of the present method, a metal salt can be suitably used if needed.

Examples of the metal salts include halides (e.g., chlorides and bromides), acetic acid salts, tetrafluoroboric acid salts, trifluoro methanesulfonic acid salts, and hexa fluorophosphoric acid salts of at least one metal selected from zinc, gold, silver, copper, lithium, sodium, potassium, cesium, magnesium, calcium, barium, iron, manganese, chromium, aluminum, indium, tin, bismuth, scandium, yttrium, zirconium, and titanium.

Of these, preferable examples include halides (e.g., chlorides and bromides) of zinc, or hexa fluorophosphoric acid salts (e.g., lithium salts, sodium salts, potassium salts) of alkali metal.

The metal salts can be used singly or in a combination of two or more.

The upper limit of the amount of the metal salt is preferably 1 mol, more preferably 0.5 mol, and even more preferably 0.25 mol, per mol of the total amount of the compounds (2a) and (2b).

The lower limit of the amount of the metal salt is preferably 0.01 mol, more preferably 0.02 mol, and even more preferably 0.05 mol, per mol of the total amount of the compounds (2a) and (2b).

The amount of the metal salt is preferably of 0.01-1 mol, more preferably 0.02-0.5 mol, and even more preferably 0.05-0.25 mol, per mol of the total amount of the compounds (2a) and (2b) .

The upper limit of the reaction temperature in step A is preferably 200°C, more preferably 125°C, and even more preferably 75°C.

The lower limit of the reaction temperature in step A is preferably -30°C, more preferably -15°C, and even more preferably 0°C.

The reaction temperature in step A is preferably -30 to 200°C, more preferably -15 to 125°C, and even more preferably 0-75°C.

An excessively low reaction temperature may cause insufficient reaction of step A.

An excessively high reaction temperature is disadvantageous in view of costs and may cause decomposition of the starting material and product, and undesirable reaction.

The upper limit of the reaction time in step A is preferably 48 hours, more preferably 24 hours, and even more preferably 12 hours.

The lower limit of the reaction time in step A is preferably 0.5 hours, and more preferably 1 hour.

The reaction time of step A is preferably 0.5-48 hours, more preferably 1-24 hours, and even more preferably 1-12 hours.

An excessively short reaction time may cause insufficient reaction of step A.

An excessively long reaction time is disadvantageous in view of costs and may cause undesirable reaction.

The reaction may be performed in the presence or absence of inert gas (e.g., nitrogen gas).

Step A can be carried out under reduced pressure, atmospheric pressure, or pressurized conditions.

Preferable examples of gases used for pressurizing include inert gases, such as nitrogen and argon; gases having reducing properties, such as carbon monoxide; and compounds of formula (1), (2a) and (2b) below and that are in a gaseous form at room temperature.

The reaction pressure is generally within -0.1 to 5 MPaG, and preferably within 0-1 MPaG.

The reaction can be performed in the presence or absence of solvent.

Examples of the solvent include aprotic solvents.

Examples of the aprotic solvent include aromatic hydrocarbons, such as benzene, toluene, and xylene; ethers, such as cyclopentyl methyl ether, tetrahydrofuran, bis(2-methoxy ethyl)ether, and 1,2-bis(2-methoxyethoxy)ethane; lactams, such as N-methylpyrrolidone; nitriles, such as acetonitrile and propionitrile; ketones, such as acetone, ethyl methyl ketone, and isobutyl methyl ketone; dialkyl sulfoxides, such as dimethyl sulfoxide; tetraalkylurea, such as 1,3-dimethyl-2-imidazolidinone, dimethylpropyleneurea, and tetramethylurea; and amides, such as N,N-dimethylformamide, N,N-dimethylacetamide, and hexaalkyl phosphoric triamide (e.g., hexa methylphosphoric acid amide).

The solvents can be used singly or in a combination of two or more.

Based on the technical common knowledge, the amount of the solvent can be determined to be an amount that is sufficient for the solvent to exhibit its function. Specifically, the amount of the solvent is preferably 0.2-100 parts by mass (pbm), more preferably 5-50 pbm, and even more preferably 10-20 pbm per pbm of the total amount of the compounds (2a) and (2b).

An excessive amount of the solvent may be disadvantageous in view of costs.

If necessary, an additive can be used in the reaction of present step A.

Examples of the additive include "hetero atom- or olefin moiety-containing compounds that are chemically neutral."

Examples of the "hetero atom- or olefin moiety-containing compounds that are chemically neutral" include ethers, such as tetrahydrofuran, bis(2-methoxyethyl)ether, and 1,2-bis(2-methoxyethoxy)ethane; nitriles, such as acetonitrile and propionitrile; ketones, such as acetone, acetophenone, and benzophenone; (bis)sulfoxides, such as dimethyl sulfoxide, diphenyl sulfoxide, and 1,2-bis(phenylsulfinyl)ethane; tetraalkylurea, such as 1,3-dimethyl-2-imidazolidinone, dimethylpropyleneurea, and tetramethylurea; amides, such as N,N-dimethylformamide, N,N-dimethylacetamide, and hexa alkyl phosphoric triamide (e.g., hexa methylphosphoric acid amide); phosphine oxides, such as triaryl phosphine oxide (e.g., triphenyl phosphine oxide) and tri(cyclo)alkylphosphine oxide (e.g., tri-tert-butylphosphine oxide); and dienes, such as cyclopentadiene, norbornadiene, and 1,5-cyclo-octadiene.

The additives can be used singly or in a combination of two or more.

The upper limit of the amount of the additive is preferably 2 mol, more preferably 1 mol, and even more preferably 0.5 mol, per mol of the total amount of the compounds (2a) and (2b) .

The lower limit of the amount of the additive is preferably 0.005 mol, more preferably 0.01 mol, and even more preferably 0.05 mol, per mol of the total amount of the compounds (2a) and (2b).

The amount of the additive is preferably 0.005-2 mol, more preferably 0.01-1 mol, and even more preferably 0.05-0.5 mol, per mol of the total amount of the compounds (2a) and (2b).

The compound of formula (1) obtained in step A was isolated or purified by a known method, such as extraction, dissolution, concentration, precipitation, dehydration, adsorption, distillation, rectification or chromatography, or combinations thereof.

According to the present method, the starting material conversion is preferably ≥ 10%, more preferably ≥ 30%, and even more preferably ≥ 50%.

According to the production method of the present invention, the selectivity of the compound of formula (1) is preferably ≥ 80%, and more preferably ≥ 90%.

According to the present method, the yield of the compound of formula (1) is preferably ≥ 50%, and more preferably ≥ 70%.

### Examples

The present invention is described in detail below with reference to Examples. In the Examples, the yield indicates GC yield unless otherwise specified.

### Example 1

288 mg of bis(dibenzylideneacetone)palladium, 262 mg of triphenylphosphine, 0.98 g of zinc powder, and 5 mL of N,N-dimethylformamide were added to a 50-mL reaction vessel. 1.16 g of chlorotrifluoroethylene was introduced thereto, and the mixture was stirred at room temperature for 24 hours.

After the completion of the reaction, gas inside the reaction vessel was collected in a cooled pressure-resistant container. The collected gas was analyzed using gas chromatography. As a result, the yields of the target hexafluorobutadiene and the remaining chlorotrifluoroethylene were respectively 5.8% and 89.3%. The yield and selectivity of trifluoroethylene were respectively 0.4% and 93.0%.

### Example 2

128 mg of bis(tri-tert-butylphosphine)palladium, 1.95 g of zinc powder, 10 mL of N,N-dimethylformamide, 10 mL of toluene, and 1 mL of dimethyl sulfoxide were added to a 50-mL reaction vessel. 0.80 g of 1-chloro-1-fluoroethylene was introduced thereto, and the mixture was stirred at 50°C for 24 hours.

After the completion of the reaction, gas inside the reaction vessel was collected in a cooled pressure-resistant container. The collected gas was analyzed using gas chromatography. As a result, the yields of the target 2,3-difluoro-1,3-butadiene and the remaining 1-chloro-1-fluoroethylene were respectively 74.7% and 22.8%. The yield and selectivity of monofluoroethylene were respectively 0.5% and 99.3%.

### Example 3

256 mg of bis(tri-tert-butylphosphine)palladium, 1.95 g of zinc powder, 10 mL of N,N-dimethylformamide, 10 mL of toluene, and 1 mL of dimethyl sulfoxide were added to a 50-mL reaction vessel. 1.16 g of chlorotrifluoroethylene was introduced thereto, and the mixture was stirred at room temperature for 6 hours.

After the completion of the reaction, gas inside the reaction vessel was collected in a cooled pressure-resistant container. The collected gas was analyzed using gas chromatography. As a result, the yields of the target hexafluorobutadiene and the remaining chlorotrifluoroethylene were respectively 86.1% and 13.0%. The yield and selectivity of trifluoroethylene were respectively 0.6% and 98.7%.

### Example 4

144 mg of bis(dibenzylideneacetone)palladium, 159 mg of di(1-adamanthyl)phosphine oxide, 0.98 g of zinc powder, 5 mL of N,N-dimethylformamide, and 1.16 g of chlorotrifluoroethylene were introduced into a 50-mL reaction vessel, and the mixture was stirred at room temperature for 24 hours.

After the completion of the reaction, gas inside the reaction vessel was collected in a cooled pressure-resistant container. The collected gas was analyzed using gas chromatography. As a result, the yields of the target hexafluorobutadiene and the remaining chlorotrifluoroethylene were respectively 12.4% and 58.7%. The yield and selectivity of trifluoroethylene were 0.5% and 96.0%.

### Example 5

1.06 g of tris{tris [3,5-bis(trifluoromethyl)phenyl]phosphine}palladium, 0.98 g of zinc powder, and 10 mL of N,N-dimethylacetamide were added to a 50-mL reaction vessel. 1.61 g of bromotrifluoroethylene was introduced thereto, and the mixture was stirred at 90°C for 24 hours.

After the completion of the reaction, gas inside the reaction vessel was collected in a cooled pressure-resistant container. The collected gas was analyzed using gas chromatography. As a result, the yields of the target hexafluorobutadiene and the remaining bromotrifluoroethylene were respectively 24.0% and 68.1%. The yield and selectivity of trifluoroethylene were respectively 0.6% and 94.4%.

### Example 6

128 mg of bis(tri-tert-butylphosphine)palladium, 1.95 g of zinc powder, 0.136 g of zinc chloride, 10 mL of N,N-dimethylformamide, and 10 mL of toluene were added to a 50-mL reaction vessel. 1.16 g of chlorotrifluoroethylene was introduced thereto, and the mixture was stirred at room temperature for 24 hours.

After the completion of the reaction, gas inside the reaction vessel was collected in a cooled pressure-resistant container. The collected gas was analyzed using gas chromatography. As a result, the yields of the target hexafluorobutadiene and the remaining chlorotrifluoroethylene were respectively 65.8% and 30.5%. The yield and selectivity of trifluoroethylene were respectively 0.5% and 95.3%.

### Example 7

288 mg of bis(dibenzylideneacetone)palladium, 268 mg of 2-(dicyclohexylphosphino)-3,6-dimethoxy- 2',4',6'-triisopropyl-1,1'-biphenyl, 0.98 g of zinc powder, and 5 mL of N,N-dimethylformamide were added to a 50-mL reaction vessel. 1.16 g of chlorotrifluoroethylene was introduced thereto, and the mixture was stirred at room temperature for 24 hours.

After the completion of the reaction, gas inside the reaction vessel was collected in a cooled pressure-resistant container. The collected gas was analyzed using gas chromatography. As a result, the yields of the target hexafluorobutadiene and the remaining chlorotrifluoroethylene were respectively 9.3% and 76.5%. The yield and selectivity of trifluoroethylene were respectively 0.3% and 95.8%.

### Example 8

144 mg of bis(dibenzylideneacetone)palladium, 101 mg of tri-tert-butylphosphine, 0.98 g of zinc powder, and 5 mL of N,N-dimethylformamide were added to a 50-mL reaction vessel. 1.16 g of chlorotrifluoroethylene was introduced thereto, and the mixture was stirred at 40°C for 6 hours.

After the completion of the reaction, gas inside the reaction vessel was collected in a cooled pressure-resistant container. The collected gas was analyzed using gas chromatography. As a result, the yields of the target hexafluorobutadiene and the remaining chlorotrifluoroethylene were respectively 39.6% and 52.4%. The yield and selectivity of trifluoroethylene were respectively 0.8% and 96.0%.

### Example 9

529 mg of tris{tris[3,5-bis(trifluoromethyl)phenyl]phosphine}palladium, 0.98 g of zinc powder, and 15 mL of N,N-dimethylformamide were added to a 50-mL reaction vessel. 1.16 g of chlorotrifluoroethylene was introduced thereto, and the mixture was stirred at 120°C for 2 hours.

After the completion of the reaction, gas inside the reaction vessel was collected in a cooled pressure-resistant container. The collected gas was analyzed using gas chromatography. As a result, the yields of the target hexafluorobutadiene and the remaining chlorotrifluoroethylene were respectively 26.8% and 36.6%. The yield and selectivity of trifluoroethylene were respectively 0.6% and 95.7%.

### Example 10

256 mg of bis(tri-tert-butylphosphine)palladium, 1.96 g of zinc powder, and 20 mL of N,N-dimethylformamide were added to a 50-mL reaction vessel. 1.16 g of chlorotrifluoroethylene was introduced thereto, and the mixture was stirred at 25°C for 8 hours.

After the completion of the reaction, gas inside the reaction vessel was collected in a cooled pressure-resistant container. The collected gas was analyzed using gas chromatography. As a result, the yields of the target hexafluorobutadiene and the remaining chlorotrifluoroethylene were respectively 69.8% and 19.7%. The yield and selectivity of trifluoroethylene were respectively 0.3% and 99.3%.

### Example 11

256 mg of bis(tri-tert-butylphosphine)palladium, 0.98 g of zinc powder, and 5 mL of N,N-dimethylformamide were added to a 50-mL reaction vessel. 1.16 g of chlorotrifluoroethylene was introduced thereto, and the mixture was stirred at 5°C for 24 hours.

After the completion of the reaction, gas inside the reaction vessel was collected in a cooled pressure-resistant container. The collected gas was analyzed using gas chromatography. As a result, the yields of the target hexafluorobutadiene and the remaining chlorotrifluoroethylene were respectively 52.1% and 36.5%. The yield and selectivity of trifluoroethylene were respectively 0.1% and 99.5%.

### Example 12

71.4 mg of dichloro(1,5-cyclooctadiene)palladium, 1.95 g of zinc powder, 10 mL of N,N-dimethylformamide, 10 mL of toluene, and 1 mL of dimethylsulfoxide were added to a 50-mL reaction vessel. 1.16 g of chlorotrifluoroethylene was introduced thereto, and the mixture was stirred at room temperature for 24 hours.

After the completion of the reaction, gas inside the reaction vessel was collected in a cooled pressure-resistant container. The collected gas was analyzed using gas chromatography. As a result, the yields of the target hexafluorobutadiene and the remaining chlorotrifluoroethylene were respectively 10.5% and 73.6%. The yield and selectivity of trifluoroethylene were respectively 0.9% and 91.9%.

### Example 13

256 mg of bis(tri-tert-butylphosphine)palladium, 1.65 g of zinc powder, 1.43 g of magnesium chloride, and 20 mL of N,N-dimethylformamide were added to a 50-mL reaction vessel. 1.16 g of chlorotrifluoroethylene was introduced thereto, and the mixture was stirred at room temperature for 8 hours.

After the completion of the reaction, gas inside the reaction vessel was collected in a cooled pressure-resistant container. The collected gas was analyzed using gas chromatography. As a result, the yields of the target hexafluorobutadiene and the remaining chlorotrifluoroethylene were respectively 11.1% and 25.6%. The yield and selectivity of trifluoroethylene were respectively 36.1% and 23.0%.

### Example 14

128 mg of bis(tri-tert-butylphosphine)palladium, 1.95 g of zinc powder, 0.682 g of zinc chloride, 10 mL of N,N-dimethylformamide, and 10 mL of tetrahydrofuran were added to a 50-mL reaction vessel. 1.16 g of chlorotrifluoroethylene was introduced thereto, and the mixture was stirred at room temperature for 22 hours.

After the completion of the reaction, gas inside the reaction vessel was collected in a cooled pressure-resistant container. The collected gas was analyzed using gas chromatography. As a result, the yields of the target hexafluorobutadiene and the remaining chlorotrifluoroethylene were respectively 61.2% and 37.4%. The yield and selectivity of trifluoroethylene were respectively 0.3% and 98.7%.

### Example 15

288 mg of bis(dibenzylideneacetone)palladium, 238 mg of 2-dicyclohexylphosphino-2',4',6'-triisopropylbiphenyl, 0.98 g of zinc powder, and 5 mL of N,N-dimethylformamide were added to a 50-mL reaction vessel. 1.16 g of chlorotrifluoroethylene was introduced thereto, and the mixture was stirred at room temperature for 24 hours.

After the completion of the reaction, gas inside the reaction vessel was collected in a cooled pressure-resistant container. The collected gas was analyzed using gas chromatography. As a result, the yields of the target hexafluorobutadiene and the remaining chlorotrifluoroethylene were respectively 9.8% and 83.1%. The yield and selectivity of trifluoroethylene were respectively 0.1% and 99.0%.

### Example 16

128 mg of bis(tri-tert-butylphosphine)palladium, 0.98 g of zinc powder, and 5 mL of N,N-dimethylacetamide were added to a 50-mL reaction vessel. 1.16 g of chlorotrifluoroethylene was introduced thereto, and the mixture was stirred at 40°C for 6 hours.

After the completion of the reaction, gas inside the reaction vessel was collected in a cooled pressure-resistant container. The collected gas was analyzed using gas chromatography. As a result, the yields of the target hexafluorobutadiene and the remaining chlorotrifluoroethylene were respectively 10.6% and 57.0%. The yield and selectivity of trifluoroethylene were respectively 0.2% and 96.5%.

### Example 17

327 mg of dichlorobis(triphenylphosphine)nickel, 0.98 g of zinc powder, and 5 mL of N,N-dimethylformamide were added to a 50-mL reaction vessel. 1.16 g of chlorotrifluoroethylene was introduced thereto, and the mixture was stirred at room temperature for 24 hours.

After the completion of the reaction, gas inside the reaction vessel was collected in a cooled pressure-resistant container. The collected gas was analyzed using gas chromatography. As a result, the yields of the target hexafluorobutadiene and the remaining chlorotrifluoroethylene were respectively 19.1% and 58.5%. The yield and selectivity of trifluoroethylene were respectively 0.7% and 95.5%.

### Example 18

61.3 mg of bis(tri-tert-butylphosphine)palladium, 1.95 g of zinc powder, 234 mg of dimethyl sulfoxide, 48.3 mg of diethylene glycol dimethyl ether, and 10 mL of N,N-dimethylformamide were added to a 50-mL reaction vessel. 1.16 g of chlorotrifluoroethylene was introduced thereto, and the mixture was stirred at room temperature for 24 hours.

After the completion of the reaction, gas inside the reaction vessel was collected in a cooled pressure-resistant container. The collected gas was analyzed using gas chromatography. As a result, the yields of the target hexafluorobutadiene and the remaining chlorotrifluoroethylene were respectively 46.5% and 51.8%. The yield and selectivity of trifluoroethylene were respectively 0.2% and 99.1%.

### Example 19

288 mg of bis(dibenzylideneacetone)palladium, 101 mg of tri-tert-butylphosphine, 0.98 g of zinc powder, and 5 mL of N,N-dimethylformamide were added to a 50-mL reaction vessel. 1.16 g of chlorotrifluoroethylene was introduced thereto, and the mixture was stirred at room temperature for 6 hours.

After the completion of the reaction, gas inside the reaction vessel was collected in a cooled pressure-resistant container. The collected gas was analyzed using gas chromatography. As a result, the yields of the target hexafluorobutadiene and the remaining chlorotrifluoroethylene were respectively 56.4% and 40.1%. The yield and selectivity of trifluoroethylene were respectively 0.6% and 98.0%.

### Example 20

327 mg of dichlorobis(triphenylphosphine)nickel, 1.95 g of zinc powder, 10 mL of N,N-dimethylformamide, 10 mL of toluene, and 1 mL of dimethylsulfoxide were added to a 50-mL reaction vessel. 0.80 g of 1-chloro-1-fluoroethylene was introduced thereto, and the mixture was stirred at 50°C for 24 hours.

After the completion of the reaction, gas inside the reaction vessel was collected in a cooled pressure-resistant container. The collected gas was analyzed using gas chromatography. As a result, the yields of the target 2,3-difluoro-1,3-butadiene and the remaining 1-chloro-1-fluoroethylene were respectively 66.9% and 21.0%. The yield and selectivity of monofluoroethylene were respectively 0.8% and 98.6%.

### Example 21

256 mg of bis(tri-tert-butylphosphine)palladium, 0.98 g of zinc powder, and 10 mL of N,N-dimethylformamide were added to a 50-mL reaction vessel. 1.16 g of chlorotrifluoroethylene was added thereto, and the mixture was stirred at room temperature for 6 hours.

After the completion of the reaction, gas inside the reaction vessel was collected in a cooled pressure-resistant container. The collected gas was analyzed using gas chromatography. As a result, the yields of the target hexafluorobutadiene and the remaining chlorotrifluoroethylene were respectively 49.6% and 27.8%. The yield and selectivity of trifluoroethylene were respectively 0.3% and 99.0%.

### Example 22

128 mg of bis(tri-tert-butylphosphine)palladium, 1.95 g of zinc powder, 1.13 g of zinc bromide, and 20 mL of N,N-dimethylformamide were added to a 50-mL reaction vessel. 1.16 g of chlorotrifluoroethylene was introduced thereto, and the mixture was stirred at room temperature for 24 hours.

After the completion of the reaction, gas inside the reaction vessel was collected in a cooled pressure-resistant container. The collected gas was analyzed using gas chromatography. As a result, the yields of the target hexafluorobutadiene and the remaining chlorotrifluoroethylene were respectively 68.1% and 27.9%. The yield and selectivity of trifluoroethylene were respectively 0.2% and 99.0%.

### Example 23

256 mg of bis(tri-tert-butylphosphine)palladium, 1.65 g of zinc powder, 1.36 g of zinc chloride, and 20 mL of N,N-dimethylformamide were added to a 50-mL reaction vessel. 1.16 g of chlorotrifluoroethylene was introduced thereto, and the mixture was stirred at room temperature for 8 hours.

After the completion of the reaction, gas inside the reaction vessel was collected in a cooled pressure-resistant container. The collected gas was analyzed using gas chromatography. As a result, the yields of the target hexafluorobutadiene and the remaining chlorotrifluoroethylene were respectively 81.6% and 5.0%. The yield and selectivity of trifluoroethylene were respectively 0.8% and 98.1%.

### Example 24

194 mg of di-µ-bromobis(tri-tert-butylphosphine) dipalladium, 1.95 g of zinc powder, and 20 mL of N,N-dimethylformamide were added to a 50-mL reaction vessel. 1.16 g of chlorotrifluoroethylene was introduced thereto, and the mixture was stirred at room temperature for 16 hours.

After the completion of the reaction, gas inside the reaction vessel was collected in a cooled pressure-resistant container. The collected gas was analyzed using gas chromatography. As a result, the yields of the target hexafluorobutadiene and the remaining chlorotrifluoroethylene were respectively 51.9% and 19.6%. The yield and selectivity of trifluoroethylene were respectively 0.2% and 99.4%.

### Example 25

256 mg of bis(tri-tert-butylphosphine)palladium, 0.98 g of zinc powder, and 5 mL of N,N-dimethylformamide were added to a 50-mL reaction vessel. After 1.16 g of chlorotrifluoroethylene was introduced thereto, nitrogen was introduced until the pressure was 0.22 MPaG. The mixture was then stirred at room temperature for 6 hours.

After the completion of the reaction, gas inside the reaction vessel was collected in a cooled pressure-resistant container. The collected gas was analyzed using gas chromatography. As a result, the yields of the target hexafluorobutadiene and the remaining chlorotrifluoroethylene were respectively 49.8% and 32.1%. The yield and selectivity of trifluoroethylene were respectively 0.1% and 99.1%.

### Example 26

61.3 mg of bis(tri-tert-butylphosphine)palladium, 1.95 g of zinc powder, 234 mg of dimethyl sulfoxide, 38.8 mg of 1,5-cyclooctadiene, 10 mL of N,N-dimethylformamide were added to a 50-mL reaction vessel. 1.16 g of chlorotrifluoroethylene was introduced thereto, and the mixture was stirred at room temperature for 21 hours.

After the completion of the reaction, gas inside the reaction vessel was collected in a cooled pressure-resistant container. The collected gas was analyzed using gas chromatography. As a result, the yields of the target hexafluorobutadiene and the remaining chlorotrifluoroethylene were respectively 47.4% and 50.0%. The yield and selectivity of trifluoroethylene were respectively 0.2% and 99.3%.

### Example 27

334 mg of bis(tricyclohexyl phosphine)palladium, 0.98 g of zinc powder, and 5 mL of N,N-dimethylformamide were added to a 50-mL reaction vessel. 1.16 g of chlorotrifluoroethylene was introduced thereto, and the mixture was stirred at room temperature for 24 hours.

After the completion of the reaction, gas inside the reaction vessel was collected in a cooled pressure-resistant container. The collected gas was analyzed using gas chromatography. As a result, the yields of the target hexafluorobutadiene and the remaining chlorotrifluoroethylene were respectively 11.3% and 79.4%. The yield and selectivity of trifluoroethylene were respectively 0.2% and 98.2%.

### Example 28

144 mg of bis(dibenzylideneacetone)palladium, 81 mg of di-tert-butylphosphine oxide, 0.98 g of zinc powder, and 5 mL of N,N-dimethylformamide were added to a 50-mL reaction vessel. 1.16 g of chlorotrifluoroethylene was introduced thereto, and the mixture was stirred at 80°C for 13 hours.

After the completion of the reaction, gas inside the reaction vessel was collected in a cooled pressure-resistant container. The collected gas was analyzed using gas chromatography. As a result, the yields of the target hexafluorobutadiene and the remaining chlorotrifluoroethylene were respectively 10.7% and 49.6%. The yield and selectivity of trifluoroethylene were respectively 0.5% and 91.2%.

### Example 29

288 mg of bis(dibenzylideneacetone)palladium, 237 mg of 1,1'-bis(di-tert-butyl phosphino)ferrocene, 0.98 g of zinc powder, and 5 mL of N,N-dimethylformamide were added to a 50-mL reaction vessel. 1.16 g of chlorotrifluoroethylene was introduced thereto, and the mixture was stirred at room temperature for 24 hours.

After the completion of the reaction, gas inside the reaction vessel was collected in a cooled pressure-resistant container. The collected gas was analyzed using gas chromatography. As a result, the yields of the target hexafluorobutadiene and the remaining chlorotrifluoroethylene were respectively 8.3% and 80.1%. The yield and selectivity of trifluoroethylene were respectively 0.2% and 97.3%.

### Example 30

1.06 g of tris{tris[3,5-bis(trifluoromethyl)phenyl]phosphine}palladium and 10 mL of toluene were added to a 50-mL reaction vessel. After 1.25 g of 1-bromo-1-fluoroethylene was introduced thereto, carbon monoxide was introduced until the pressure was 0.5 MPaG. The mixture was then stirred at 90°C for 24 hours.

After the completion of the reaction, gas inside the reaction vessel was collected in a cooled pressure-resistant container. The collected gas was analyzed using gas chromatography. As a result, the yields of the target 2,3-difluoro-1,3-butadiene and the remaining 1-bromo-1-fluoroethylene were respectively 35.7% and 10.7%. The yield and selectivity of monofluoroethylene were respectively 0.4% and 98.1%.

### Example 31

289 mg of tetrakis(triphenylphosphine)palladium, 1.95 g of zinc powder, 10 mL of N,N-dimethylformamide, 10 mL of toluene, and 1 mL of dimethylsulfoxide were added to a 50-mL reaction vessel. 0.80 g of 1-chloro-1-fluoroethylene was introduced thereto, and the mixture was stirred at room temperature for 36 hours.

After the completion of the reaction, gas inside the reaction vessel was collected in a cooled pressure-resistant container. The collected gas was analyzed using gas chromatography. As a result, the yields of the target 2,3-difluoro-1,3-butadiene and the remaining 1-chloro-1-fluoroethylene were respectively 10.1% and 77.6%. The yield and selectivity of monofluoroethylene were respectively 0.4% and 96.2%.

### Example 32

128 mg of bis(tri-tert-butylphosphine)palladium, 0.98 g of zinc powder, and 5 mL of dimethyl sulfoxide were added to a 50-mL reaction vessel. 1.16 g of chlorotrifluoroethylene was introduced thereto, and the mixture was stirred at 20°C for 18 hours.

After the completion of the reaction, gas inside the reaction vessel was collected in a cooled pressure-resistant container. The collected gas was analyzed using gas chromatography. As a result, the yields of the target hexafluorobutadiene and the remaining chlorotrifluoroethylene were respectively 29.0% and 52.8%. The yield and selectivity of trifluoroethylene were respectively 0.4% and 97.3%.

### Example 33

256 mg of bis(tri-tert-butylphosphine)palladium, 0.98 g of zinc powder, and 10 mL of N,N-dimethylformamide were added to a 50-mL reaction vessel. 1.16 g of chlorotrifluoroethylene was introduced thereto, and the mixture was stirred at 60°C for 1.5 hours.

After the completion of the reaction, gas inside the reaction vessel was collected in a cooled pressure-resistant container. The collected gas was analyzed using gas chromatography. As a result, the yields of the target hexafluorobutadiene and the remaining chlorotrifluoroethylene were respectively 53.1% and 25.2%. The yield and selectivity of trifluoroethylene were respectively 0.3% and 99.0%.

### Example 34

128 mg of bis(tri-tert-butylphosphine)palladium, 0.98 g of zinc powder, and 5 mL of N-methylpyrrolidone were added to a 50-mL reaction vessel. 1.16 g of chlorotrifluoroethylene was introduced thereto, and the mixture was stirred at room temperature for 6 hours.

After the completion of the reaction, gas inside the reaction vessel was collected in a cooled pressure-resistant container. The collected gas was analyzed using gas chromatography. As a result, the yields of the target hexafluorobutadiene and the remaining chlorotrifluoroethylene were respectively 18.4% and 68.7%. The yield and selectivity of trifluoroethylene were respectively 0.8% and 92.3%.

### Example 35

128 mg of bis(tri-tert-butylphosphine)palladium, 0.98 g of zinc powder, and 15 mL of N,N-dimethylformamide were added to a 50-mL reaction vessel. 1.16 g of chlorotrifluoroethylene was introduced thereto, and the mixture was stirred at 60°C for 1.5 hours.

After the completion of the reaction, gas inside the reaction vessel was collected in a cooled pressure-resistant container. The collected gas was analyzed using gas chromatography. As a result, the yields of the target hexafluorobutadiene and the remaining chlorotrifluoroethylene were respectively 58.0% and 30.9%. The yield and selectivity of trifluoroethylene were respectively 0.5% and 99.1%.

### Example 36

327 mg of dichlorobis(triphenylphosphine)nickel, 1.95 g of zinc powder, 10 mL of N,N-dimethylformamide, 10 mL of toluene, and 1 mL of dimethyl sulfoxide were added to a 50-mL reaction vessel. 0.80 g of 1-chloro-1-fluoroethylene was introduced thereto, and the mixture was stirred at room temperature for 24 hours.

After the completion of the reaction, gas inside the reaction vessel was collected in a cooled pressure-resistant container. The collected gas was analyzed using gas chromatography. As a result, the yields of the target 2,3-difluoro-1,3-butadiene and the remaining 1-chloro-1-fluoroethylene were respectively 21.8% and 72.7%. The yield and selectivity of monofluoroethylene were respectively 0.3% and 98.6%.

### Example 37

128 mg of bis(tri-tert-butylphosphine)palladium, 1.95 g of zinc powder, 0.92 g of potassium hexafluorophosphorate, and 20 mL of N,N-dimethylformamide were added to a 50-mL reaction vessel. 1.16 g of chlorotrifluoroethylen was introduced thereto, and the mixture was stirred at room temperature for 24 hours.

After the completion of the reaction, gas inside the reaction vessel was collected in a cooled pressure-resistant container. The collected gas was analyzed using gas chromatography. As a result, the yields of the target hexafluorobutadiene and the remaining chlorotrifluoroethylene were respectively 61.9% and 26.9%. The yield and selectivity of trifluoroethylene were respectively 0.2% and 89.0%.

### Example 38

144 mg of bis(dibenzylideneacetone)palladium, 145 mg of tri-tert-butylphosphine tetrafluoroborate, 212 mg of potassium phosphate, 0.98 g of zinc powder, and 5 mL of N,N-dimethylformamide were added to a 50-mL reaction vessel. 1.16 g of chlorotrifluoroethylene was introduced thereto, and the mixture was stirred at 40°C for 6 hours.

After the completion of the reaction, gas inside the reaction vessel was collected in a cooled pressure-resistant container. The collected gas was analyzed using gas chromatography. As a result, the yields of the target hexafluorobutadiene and the remaining chlorotrifluoroethylene were respectively 31.2% and 53.4%. The yield and selectivity of trifluoroethylene were respectively 0.5% and 96.6%.

### Example 39

256 mg of bis(tri-tert-butylphosphine)palladium, 1.65 g of manganese powder, and 20 mL of N,N-dimethylformamide were added to a 50-mL reaction vessel. 1.16 g of chlorotrifluoroethylene was introduced thereto, and the mixture was stirred at room temperature for 8 hours.

After the completion of the reaction, gas inside the reaction vessel was collected in a cooled pressure-resistant container. The collected gas was analyzed using gas chromatography. As a result, the yields of the target hexafluorobutadiene and the remaining chlorotrifluoroethylene were respectively 27.7% and 37.2%. The yield and selectivity of trifluoroethylene were respectively 0.5% and 96.7%.

### Example 40

61.3 mg of bis(tri-tert-butylphosphine)palladium, 1.95 g of zinc powder, 234 mg of dimethyl sulfoxide, and 10 mL of N,N-dimethylformamide were added to a 50-mL reaction vessel. 1.16 g of chlorotrifluoroethylene was introduced thereto, and the mixture was stirred at room temperature for 24 hours.

After the completion of the reaction, gas inside the reaction vessel was collected in a cooled pressure-resistant container. The collected gas was analyzed using gas chromatography. As a result, the yields of the target hexafluorobutadiene and the remaining chlorotrifluoroethylene were respectively 53.0% and 41.0%. The yield and selectivity of trifluoroethylene were respectively 0.4% and 98.4%.

### Example 41

128 mg of bis(tri-tert-butylphosphine)palladium, 1.95 g of zinc powder, 10 mL of N,N-dimethylformamide, 10 mL of toluene, and 1 mL of dimethyl sulfoxide were added to a 50-mL reaction vessel. 1.31 g of 2-chloro-3,3,3-trifluoropropene was introduced thereto, and the mixture was stirred at 80°C for 18 hours.

After the completion of the reaction, gas inside the reaction vessel was collected in a cooled pressure-resistant container. The collected gas was analyzed using gas chromatography. As a result, the yields of the target 2,3-bis(trifluoromethyl)-1,3-butadiene and the remaining 2-chloro-3,3,3-trifluoropropene were respectively 25.3% and 62.7%. The selectivity was 100%.

### Example 42

25.6 mg of bis(tri-tert-butylphosphine)palladium, 0.39 g of zinc powder, 2 mL of N,N-dimethylformamide, 2 mL of toluene, and 0.2 mL of dimethyl sulfoxide were added to a 50-mL reaction vessel. 400 mg of β-bromo-β-fluorostyrene was introduced thereto, and the mixture was stirred at 80°C for 18 hours.

After the completion of the reaction, the cooled reaction liquid was quantified by ¹⁹F NMR. As a result, the yields of the target 2,3-difluoro-1,4-diphenyl-1,3-butadiene and the remaining β-bromo-β-fluorostyrene were respectively 44.0% and 14.7%. The yield and selectivity of β-fluorostyrene were respectively 23.5% and 65.2%.

## Claims

1. A method for producing a compound of formula (1): 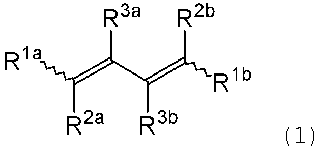 wherein R^{1a}, R^{1b}, R^{2a}, R^{2b}, R^{3a}, and R^{3b} each independently are H, F, alkyl, fluoroalkyl or aryl; provided that at least one of them is F or fluoroalkyl; or one or more moieties among R^{1a}-C-R^{2a}, R^{1b}-C-R^{2b}, R^{1a}-C=C-R^{3a} and R^{1b}-C=C-R^{3b} each independently may form a non-aromatic carbon ring that may be substituted with one or more fluorine atoms; and
each single bond expressed by a wavy line shows that the configuration with respect to a double bond to which the single bond is bonded is E configuration, Z configuration, or a mixture thereof at any ratio;
the method comprising step A of subjecting a compound of formula (2a): 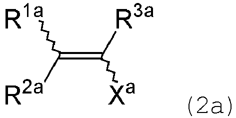 wherein X^{a} is Cl, Br or I, and R^{1a}-R^{3a} are as defined above, and a compound of formula (2b): 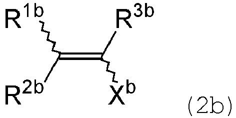 wherein X^{b} is Cl, Br or I, and R^{1b}-R^{3b} are as defined above, to a coupling reaction in the presence of
(1) a metal catalyst which is at least one of nickel catalysts, palladium catalysts, platinum catalysts, ruthenium catalysts, rhodium catalysts, and cobalt catalysts, and
(2) one or more reducing agents selected from (a) transition metal elements in the 4th period, (b) metal elements in group 12, (c) metal elements in group 13 and (e) carbon monoxide.

2. The method of claim 1, wherein in formula (1), and correspondingly in the formulae (2a) and (2b), either or both of R^{1a}-C-R^{2a} and R^{1b}-C-R^{2b} each independently may form a non-aromatic carbon ring that may be substituted with one or more fluorine atoms.

3. The method of claim 1, wherein in formula (1), and correspondingly in the formulae (2a) and (2b),
R^{1a} is H, F, alkyl, fluoroalkyl or aryl,
R^{2a} is H, F or fluoroalkyl; or R^{1a}-C-R^{2a} may form a non-aromatic carbon ring that may be substituted with one or more fluorine atoms;
R^{3a} is F or fluoroalkyl;
R^{1b} is H, F, alkyl, fluoroalkyl or aryl, and
R^{2b} is H, F or fluoroalkyl; or R^{1b}-C-R^{2b} may form a non-aromatic carbon ring that may be substituted with one or more fluorine atoms; and
R^{3b} is F or fluoroalkyl.

4. The method of any of claims 1-3, wherein R^{1a} is H, F, trifluoromethyl or aryl.

5. The method of any of claims 1-4, wherein R^{2a} is H, F or trifluoromethyl.

6. The method of any of claims 1-5, wherein R^{3a} is F or trifluoromethyl.

7. The method of any of claims 1-6, wherein R^{1b} is H, F, trifluoromethyl or aryl.

8. The method of any of claims 1-7, wherein R^{2b} is H, F or trifluoromethyl.

9. The method of any of claims 1-8, wherein R^{3b} is F or trifluoromethyl.

10. The method of any of claims 1-9, wherein X^{a} is Br or Cl.

11. The method of any of claims 1-10, wherein X^{b} is Br or Cl.

12. The method of any of claims 1-11, wherein the compounds of formulae (2a) and (2b) are the same.

13. The method of any of claims 1-12, wherein the metal catalyst is a palladium catalyst or a nickel catalyst.

14. The method of claim 13, wherein the metal catalyst is a palladium catalyst or a nickel catalyst, and a ligand of the metal catalyst is at least one of dialkyl monoaryl phosphines, diaryl monoalkyl phosphines, trialkylphosphines, triarylphosphines, and bidentate diphosphines.

15. The method of any of claims 1-14, wherein the reducing agent is at least one of titanium, vanadium, chromium, manganese, iron, cobalt, copper, zinc, cadmium, mercury, aluminum, gallium, and indium.

## Patentansprüche

1. Verfahren zur Herstellung einer Verbindung der Formel (1) : 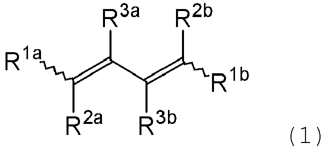 worin R^{1a}, R^{1b}, R^{2a}, R^{2b}, R^{3a} und R^{3b} jeweils unabhängig für H, F, Alkyl, Fluoralkyl oder Aryl stehen; mit der Maßgabe, dass mindestens eines davon für F oder Fluoralkyl steht; oder eine oder mehrere Einheiten aus R^{1a}-C-R^{2a}, R^{1b}-C-R^{2b}, R^{1a}-C=C-R^{3a} und R^{1b}-C=C-R^{3b} jeweils unabhängig einen nicht-aromatischen Kohlenstoffring bilden können, der mit einem oder mehreren Fluoratomen substituiert sein kann; und
jede durch eine gewellte Linie wiedergegebene Einfachbindung anzeigt, dass die Konfiguration der Doppelbindung, mit der die Einfachbindung verbunden ist, in E-Konfiguration, Z-Konfiguration oder einem Gemisch davon in jeglichem Verhältnis vorliegt;
das Verfahren umfassend Schritt A, bei dem eine Verbindung der Formel (2a): 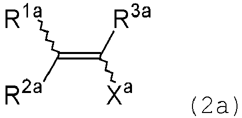 worin X^{a} für Cl, Br oder I steht und R^{1a}-R^{3a} wie oben definiert sind, und eine Verbindung der Formel (2b): 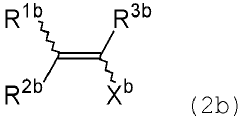 worin X^{b} für Cl, Br oder I steht und R^{1b}-R^{3b} wie oben definiert sind,
einer Kupplungsreaktion in Gegenwart von
(1) einem Metallkatalysator, der mindestens einer ist aus Nickelkatalysatoren, Palladiumkatalysatoren, Platinkatalysatoren, Rutheniumkatalysatoren, Rhodiumkatalysatoren und Cobaltkatalysatoren, und
(2) einem oder mehreren Reduktionsmitteln, ausgewählt aus (a) Übergangsmetallelementen der 4. Periode, (b) Metallelementen der Gruppe 12, (c) Metallelementen der Gruppe 13 und (e) Kohlenmonoxid,
unterzogen werden.

2. Verfahren gemäß Anspruch 1, worin in Formel (1), und dementsprechend in den Formeln (2a) und (2b), entweder eines oder beides aus R^{1a}-C-R^{2a} und R^{1b}-C-R^{2b} jeweils unabhängig einen nicht-aromatischen Kohlenstoffring bilden kann, der mit einem oder mehreren Fluoratomen substituiert sein kann.

3. Verfahren gemäß Anspruch 1, worin in Formel (1), und dementsprechend in den Formeln (2a) und (2b),
R^{1a} für H, F, Alkyl, Fluoralkyl oder Aryl steht,
R^{2a} für H, F oder Fluoralkyl steht; oder R^{1a}-C-R^{2a} einen nicht-aromatischen Kohlenstoffring bilden kann, der mit einem oder mehreren Fluoratomen substituiert sein kann;
R^{3a} für F oder Fluoralkyl steht;
R^{1b} für H, F, Alkyl, Fluoralkyl oder Aryl steht und
R^{2b} für H, F oder Fluoralkyl steht; oder R^{1b}-C-R^{2b} einen nicht-aromatischen Kohlenstoffring bilden kann, der mit einem oder mehreren Fluoratomen substituiert sein kann; und
R^{3b} für F oder Fluoralkyl steht.

4. Verfahren gemäß einem der Ansprüche 1-3, worin R^{1a} für H, F, Trifluormethyl oder Aryl steht.

5. Verfahren gemäß einem der Ansprüche 1-4, worin R^{2a} für H, F oder Trifluormethyl steht.

6. Verfahren gemäß einem der Ansprüche 1-5, worin R^{3a} für F oder Trifluormethyl steht.

7. Verfahren gemäß einem der Ansprüche 1-6, worin R^{1b} für H, F, Trifluormethyl oder Aryl steht.

8. Verfahren gemäß einem der Ansprüche 1-7, worin R^{2b} für H, F oder Trifluormethyl steht.

9. Verfahren gemäß einem der Ansprüche 1-8, worin R^{3b} für F oder Trifluormethyl steht.

10. Verfahren gemäß einem der Ansprüche 1-9, worin X^{a} für Br oder Cl steht.

11. Verfahren gemäß einem der Ansprüche 1-10, worin X^{b} für Br oder Cl steht.

12. Verfahren gemäß einem der Ansprüche 1-11, worin die Verbindungen der Formeln (2a) und (2b) dieselben sind.

13. Verfahren gemäß einem der Ansprüche 1-12, worin der Metallkatalysator ein Palladiumkatalysator oder ein Nickelkatalysator ist.

14. Verfahren gemäß Anspruch 13, worin der Metallkatalysator ein Palladiumkatalysator oder ein Nickelkatalysator ist und der Ligand des Metallkatalysators mindestens einer ist aus Dialkylmonoarylphosphinen, Diarylmonoalkylphosphinen, Trialkylphosphinen, Triarylphosphinen und bidentaten Diphosphinen.

15. Verfahren gemäß einem der Ansprüche 1-14, worin das Reduktionsmittel mindestens eines ist aus Titan, Vanadium, Chrom, Mangan, Eisen, Cobalt, Kupfer, Zink, Cadmium, Quecksilber, Aluminium, Gallium und Indium.

## Revendications

1. Procédé de production d'un composé de formule (1) : 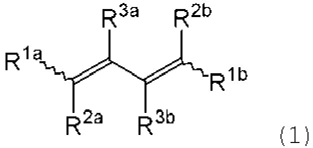 dans lequel R^{1a}, R^{1b}, R^{2a}, R^{2b}, R^{3a} et R^{3b} sont chacun indépendamment H, F, un alkyle, un fluoroalkyle ou un aryle ; à condition qu'au moins un d'entre eux soit F ou un fluoroalkyle ; ou un ou plusieurs fragments parmi R^{1a}-C-R^{2a}, R^{1b}-C-R^{2b}, R^{1a-}C=C-R^{3a} et R^{1b}-C=C-R^{3b} peuvent former chacun indépendamment un cycle carboné non aromatique qui peut être substitué par un ou plusieurs atomes de fluor ; et chaque liaison simple exprimée par une ligne ondulée montre que la configuration par rapport à une liaison double à laquelle la liaison simple est liée est une configuration E, une configuration Z, ou un mélange de celles-ci à un rapport quelconque ;
le procédé comprenant l'étape A de soumission d'un composé de formule (2a) : 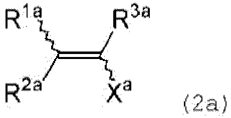 dans lequel X^{a} est Cl, Br ou I, et R^{1a}-R^{3a} sont tels que définis ci-dessus, et un composé de formule (2b) : 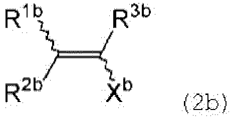 dans lequel X^{b} est Cl, Br ou I, et R^{1b}-R^{3b} sont tels que définis ci-dessus, à une réaction de couplage en présence de
(1) un catalyseur métallique qui est au moins un parmi des catalyseurs au nickel, des catalyseurs au palladium, des catalyseurs au platine, des catalyseurs au ruthénium, catalyseurs au rhodium et des catalyseurs au cobalt, et
(2) un ou plusieurs agents réducteurs sélectionnés parmi (a) des éléments métalliques de transition de la 4e période, (b) des éléments métalliques du groupe 12, (c) des éléments métalliques du groupe 13 et (e) du monoxyde de carbone.

2. Procédé selon la revendication 1, dans lequel dans la formule (1), et de manière correspondante dans les formules (2a) et (2b), l'un ou l'autre des R^{1a}-C-R^{2a} et R^{1b}-C-R^{2b} ou les deux peuvent chacun indépendamment former un cycle carboné non aromatique qui peut être substitué par un ou plusieurs atomes de fluor.

3. Procédé selon la revendication 1, dans lequel dans la formule (1), et de manière correspondante dans les formules (2a) et (2b),
R^{1a} est H, F, un alkyle, un fluoroalkyle ou un aryle,
R^{2a} est H, F ou un fluoroalkyle ; ou R^{1a}-C-R^{2a} peut former un cycle carboné non aromatique qui peut être substitué par un ou plusieurs atomes de fluor ;
R^{3a} est F ou un fluoroalkyle ;
R^{1b} est H, F, un alkyle, un fluoroalkyle ou un aryle, et
R^{2b} est H, F ou un fluoroalkyle ; ou R^{1b}-C-R^{2b} peut former un cycle carboné non aromatique qui peut être substitué par un ou plusieurs atomes de fluor ; et
R^{3b} est F ou un fluoroalkyle.

4. Procédé selon l'une quelconque des revendications 1 à 3, dans lequel R^{1a} est H, F, un trifluorométhyle ou un aryle.

5. Procédé selon l'une quelconque des revendications 1 à 4, dans laquelle R^{2a} est H, F ou un trifluorométhyle.

6. Procédé selon l'une quelconque des revendications 1 à 5, dans lequel R^{3a} est F ou un trifluorométhyle.

7. Procédé selon l'une quelconque des revendications 1 à 6, dans lequel R^{1b} est H, F, un trifluorométhyle ou un aryle.

8. Procédé selon l'une quelconque des revendications 1 à 7, dans lequel R^{2b} est H, F ou un trifluorométhyle.

9. Procédé selon l'une quelconque des revendications 1 à 8, dans lequel R^{3b} est F ou un trifluorométhyle.

10. Procédé selon l'une quelconque des revendications 1 à 9, dans lequel X^{a} est Br ou Cl.

11. Procédé selon l'une quelconque des revendications 1 à 10, dans lequel X^{b} est Br ou Cl.

12. Procédé selon l'une quelconque des revendications 1 à 11, dans lequel les composés de formules (2a) et (2b) sont les mêmes.

13. Procédé selon l'une quelconque des revendications 1 à 12, dans lequel le catalyseur métallique est un catalyseur au palladium ou un catalyseur au nickel.

14. Procédé selon la revendication 13, dans lequel le catalyseur métallique est un catalyseur au palladium ou un catalyseur au nickel, et un ligand du catalyseur métallique est au moins un élément parmi des dialkyl monoaryl phosphines, des diaryl monoalkyl phosphines, des trialkylphosphines, des triarylphosphines et des diphosphines bidentées.

15. Procédé selon l'une quelconque des revendications 1 à 14, dans lequel l'agent réducteur est au moins un parmi le titane, le vanadium, le chrome, le manganèse, le fer, le cobalt, le cuivre, le zinc, le cadmium, le mercure, l'aluminium, le gallium et l'indium.
